# EUROPEAN PATENT SPECIFICATION

(11) **EP 0 970 245 B1**
(45) Date of publication and mention of the grant of the patent: **01.12.2004**
(21) Application number: 98910267.8
(22) Date of filing: 09.03.1998
(51) Int. Cl.: C12Q 1/68

(54) **METHOD FOR THE IDENTIFICATION OF GENETIC SUBTYPES**
VERFAHREN ZUR IDENTIFIZIERUNG VON GENETISCHEN SUBTYPEN
PROCEDE D'IDENTIFICATION DE SOUS-TYPES GENETIQUES

(30) Priority: 10.03.1997 US 39056 P
(43) Date of publication of application: 12.01.2000
(73) Proprietor: Samadpour, Mansour, Seattle, WA 98115 (US)
(72) Inventor: Samadpour, Mansour, Seattle, WA 98115 (US)
(74) Representative: Webber, Philip Michael
(86) International application number: PCT/US1998/004599
(87) International publication number: WO 1998/040515

(56) References cited:
- EP-A- 0 266 787
- SAMADPOUR M ET AL: "MOLECULAR EPIDEMIOLOGY OF ESCHERICHIA COLI O157:H7 BY RESTRICTION FRAGMENT LENGTH POLYMORPHISM USING SHIGA-LIKE TOXIN GENES" JOURNAL OF CLINICAL MICROBIOLOGY, vol. 33, no. 8, August 1995, pages 2150-2154, XP000197545
- LI H ET AL: "Multiplex genotype determination at a DNA sequence polymorphism cluster in the human immunoglobulin heavy-chain region." GENOMICS, vol. 26, no. 2, 20 March 1995, SAN DIEGO, pages 199-206, XP002068684
- GRIMM LM ET AL: "Molecular epidemiology of a fast-food restaurant-associated outbreak of Escherichia coli O157:H7 in Washington State." JOURNAL OF CLINICAL MICROBIOLOGY, vol. 33, no. 8, August 1995, WASHINGTON, DC, pages 2155-2158, XP002068685
- FERNANDEZ-PERALTA AM ET AL: "Digestion of human chromosomes by means of the isoschizomers MspI and HpaII." GENOME, vol. 37, no. 5, October 1994, OTTAWA, pages 770-774, XP002068686
- PRESTON ET AL.: "Genetic variability and molecular typing of Shigella sonnei strains isolated in Canada" JOURNAL OF CLINICAL MICROBIOLOGY, vol. 32, no. 6, June 1994, WASHINGTON, DC, pages 1427-1430, XP002068687
- HAERTL ET AL.: "Use of small fragment restriction endonuclease analysis (SF-REA) for epidemiological fingerprinting of Klebsiella oxytoca" ZENTRALBLATT FÜR BAKTERIOLOGY, vol. 280, no. 8, January 1994, pages 312-318, XP002068688

## Description

### FIELD OF INVENTION

The present invention relates to the field of microbiology and methods for identification of genetic subtypes within a given genetic population. More specifically the process involves the method of Restriction Fragment Length Polymorphism (RFLP) using frequent cutting restriction endonucleases (RE) to generate a genetic fingerprint distinctive of a specific genetic subtype.

### BACKGROUND

Infectious diseases are a prominent health hazard in industrial as well as developing countries. For example, two to three million cases of *Salmonella* food poisoning are estimated to occur each year in the United States. Acute symptoms of this disorder include nausea, vomiting, diarrhea, cold chills, fever, and exhaustion. About 2,000-3,000 (0.1%) people die each year in the United States from *Salmonella* poisoning, these victims usually including infants, the sick, and elderly.

Poultry, red meat, seafood, eggs and any foods which contain these products particularly have the potential to carry bacterial pathogens. The challenge to an epidemiologist dealing with infectious diseases, is to be able to discern, not only which pathogen is responsible for the disease, but also which serotype or subtype of the pathogen is present. The ability to subtype the organisms causing a particular disease offers the best chance of determining the source of the outbreak and controlling the spread of the disease.

As methods of molecular subtyping expand in epidemiological investigations of infectious diseases there is a corresponding need for universal subtyping methods. Several methods of genetic subtyping are practiced, including macro-restriction fragment length polymorphism (Ma-RFLP) and PCR based methods.

Ma-RFLP methods involve the use of restriction endonucleases that cut DNA infrequently (rare cutters) which results in the generation of a discreet number of relatively large DNA fragments. These fragments are resolved by gel electrophoresis where the banding pattern on the gel is used to differentiate discrete strains and subtypes within a population of microorganisms. The DNA fragment size produced in Ma-RFLP is generally on the order of 10-1000 kilobase pairs (kb).

Ma-RFLP has been used effectively in epidemiological studies for the subtyping of organisms. For example, the method has been used to differentiate strains and subtypes of *Listeria* [Nesbakken et al., *International Journal of Food Microbiology* 31 (1-3), 1996, 161-171; Saito et al., *Japanese Journal of Medical Science and Biology* 50 (2), 1997, 63 -71.; Howard et al., *Appl. Environ. Microbiol.* (1992), 58(2), 709-12;] *Salmonella,* [Kapperud et al., *J Clin Microbiol* 27 (9), 1989, 2019-2024.; Koh-Luar et al., *World Journal of* *Microbiology & Biotechnology* 12 (4), 1996, 405-407] *Campylobacter,* [Owen et al., *J Clin Microbiol* 27 (10), 1989, 2338-2343], and *Pseudomonas* [Goldstein, R., WO 9701647].

Although Ma-RFLP is the current method of choice for subtype determination the method is labor-intensive, cumbersome, takes 2-5 days to perform, and requires expensive electrophoretic equipment such as pulsed field gel electrophoresis(PFGE) apparatus for resolving the DNA fragments. Additionally the resolution of gel bands is often poor, making interpretation of the data suspect.

Another commonly used method of genetic subtyping employs polymerase chain reaction (PCR) amplification of geneomic or chromosomal DNA either alone or in combination with restriction analysis or probe hybridization. Typically PCR primers, specific for a particular organism, are used to amplify a specific portion of the genome, which is then restricted using appropriate restriction enzymes. For example PCR based genetic subtyping has been used to analysis of populations of *Mycobacterium*, [Haas et al., *Journal of Clinical Microbiology* 35 (3), 1997, 663-666, (epidemiological tracking of *M africanum and M tuberculosis*)], and *Salmonella,* [Burr et al., *J. Microbiol. Methods* (1997), 29(1), 63-68, (distinguishing *Salmonella* and *E*. *coli* serotypes); Lin et al., *J*. *Clin. Microbiol.* (1996), 34(4), 870-6, (differentiation of *S*. *enteritidis* isolates].

PCR based methods, while effective in differentiating some genetic subtypes, generally require advanced knowledge of the genome for the generation of specific primers, making the method less applicable to situations where little is known about the subject organism.

A method termed small fragment restriction endonuclease analysis (SF-REA) has been developed and has been used for epidemiological studies [Haertl et al., *Zentralbl. Bakteriol.* (1994), 280(3), 312-18]. This technique relies on the use of infrequent cutting restriction endonucleases and analyzes the fragments generated in the 0.5 to 4 kb range. Restriction enzymes with 6-base recognition sequences are used to generate the RFLP pattern. The RFLP pattern range starts at 20-40 kb, and is virtually impossible to decipher. Polyacrylamide gel separation is necessary to achieve effective separation in the lower size range which has very limited use for interstrain differentiation.

Yet another method for genetic subtyping has been developed involving the application of restriction analysis to chromosomal DNA. The method is termed chromosomal restriction endonuclease analysis (CREA) and has been applied to various prokaryotic genomes with only moderate success. For example, Tsai et al., *(Gyobyo Kenkyu* (1990), 25(4), 201-6) disclose the restriction of genomic DNA isolated from the fish pathogens *Vibrio alginolyticus, V*. *anguillarum, V*. *damsela, V*. *harveyi, V*. *ordalii, V*. *parahaemolyticus*, and *V*. *vulnificus* with a variety of restriction enzymes and resolution of fragments ranging from 2 kb to 23 kb by gel electrophoresis to obtain a characteristic fingerprint for each pathogen. Only 6 base specific restriction endonucleases were effective in generating specific patterns.

For the most part RFLP subtyping has employed rare cutters (infrequent cutters) in order to generate a manageable number of fragments. There are a few reports where more frequent cutters are used, with minimal success. For example, Preston et al. (*J*. *Clin*. *Microbiol.* (1994), 32(6), 1427-30) describe a method for the RFLP analysis of 49 clinical isolates of *Shigella sonnei* with the four base cutting restriction endonucleases *HaeIII* and *RsaI*. Restriction fragments were resolved by gel electrophoresis and visualized by silver staining. Analysis revealed 15 distinct RFLP patterns by *HaeIII* digestion, and 12 distinct patterns by *RsaI* digestion. The authors note that it was not possible to detect the bands on the gel using a DNA binding dye and silver stain was substituted as a detection means. Additionally, polyacrylamide was taught as the only effective gel medium for this type of separation.

Similarly, Wesley, et al., (*Microb*. *Toxins Foods Feeds:* Cell. Mol. Modes Action, [Proc. Symp.] (1990), Meeting Date 1988, 225-38. Editor(s): Pohland, Albert E.; Dowell, Vulus R., Jr.; Richard, John L. Publisher: Plenum, New York, NY) teach the restriction of genomic DNA isolated from *L. monocytogenes* with the four base cutter *HhaI* and resolution of the resulting fragments by gel electrophoresis. Although a frequent base cutter was used, Southern blotting and hybridization with a synthetic oligomer probe specific for the beta-hemolysin gene was necessary for final resolution.

Like Wesley et al., Matsuura, et al., (*J*. *Helminthol.* (1992), 66(4), 261-6) teach the use of seven kinds of restriction endonucleases including 4-base cutters for the digestion of *Diphyllobothrium* ribosomal DNA and resolution of the fragments on a gel for the identification of species-specific patterns. Final differentiation of subtypes was made using a characteristic 25S rDNA probe. This method of subtyping by Southern blot hybridization with ribosomal RNA-based probes, commonly referred to as ribotyping, is well known in the scientific literature.

The above mentioned methods are useful for genetic subtyping but are limited by lack of specificity and efficiency of application. There is a need therefore for a method that provides the rapid, specific and accurate subtyping of genetic populations that does not rely on the cumbersome techniques of Ma-RFLP pulsed-field gel analysis.

The Applicant has provided the solution to the stated problem by developing a highly specific restriction enzyme based process that analyzes the DNA banding patterns of small DNA fragments produced by the use of appropriate restriction enzymes (mostly frequent base cutters) which yield banding patterns in the size range below 18 kb.

### SUMMARY OF THE INVENTION

The present invention provides a method for the differentiation of genetic sub-types within a population of genetically related organisms comprising the steps of:
(i) isolating genomic DNA from an organism to be analyzed;
(ii) restricting an excess of genomic DNA from said organism with at least one restriction endonuclease to yield restricted DNA having fragments less than 18 kb;
(iii) resolving said restricted DNA by chromatographic means to generate a distinct genetic fingerprint;
(iv) comparing said distinct genetic fingerprint of step (iii) with the genetic fingerprint of a specific genetic sub-type wherein differences in said genetic fingerprints afford differentiation of genetic sub-types within a population of genetically related organisms.

The invention further provides a method for the interstrain differentiation of bacterial isolates, comprising the steps of:
(i) isolating genomic DNA from a) a panel of epidemiologically linked bacterial isolates and b) a panel of epidemiologically un-linked bacterial isolates wherein said linked and un-linked isolates are members of the same genus;
(ii) restricting an excess of genomic DNA from the linked and un-linked panel of isolates with a panel of restriction enzymes selected from the group consisting of *AccI*, *AluI, AvaI*, *AvaII, BanII, BfaI, CfoI, DdeI, DpnI, HaeII, HaeIII, HhaI, HincII*, *HinfI*, HpaII, *MboI*, *MnII*, *MseI*, *NciI*, *MaIV*, RsaI, *Sau3AI*, *ScrfI*, *TaqI*, and *ThaI*;
(iii) resolving the restricted DNA of step (ii) by gel electrophoresis to generate a banding pattern for each enzyme;
(iv) selecting at least one enzyme from the panel of enzymes of step (ii) that a) generates DNA fragments less than 18 kb, b) generates the lowest index of diversity from the linked panel and c) generates the highest index of diversity from the unlinked panel;
(v) restricting the isolated DNA from the linked and un-linked panels of bacterial isolates of step (i) with the at least one enzyme of step (iv);
(vi) resolving said restricted DNA of step (v) by gel electrophoresis to generate a distinct genetic fingerprint for said linked and unlinked isolates; and
(vii) comparing said distinct genetic fingerprints of step (vi) wherein differences in said genetic fingerprints affords interstrain differentiation of said bacterial isolates.

### DETAILED DESCRIPTION OF THE INVENTION

As used herein the following terms may be used for interpretation of the claims and specification.

The term "genetic subtype" means an individual microbial cell or group of microbial cells of a particular taxonomic genus, species, subspecies, serovar or strain which can be distinguished on the basis of differences in genomic DNA sequence or structure revealed by DNA sequencing, restriction fragment length polymorphism or other DNA-based methodology from other individual microbial cells or groups of microbial cells of the same taxonomic classification.

The terms "genetic subtyping" and "inter-strain differentiation" will be used interchangeably and will refer to any process for identifying a genetic subtype.

The term "epidemiological history" means information which relates microbial strains isolated from clusters of patients grouped on the basis of common exposure to a specific microbe. Discrete microbial isolates which have a known epidemiological history may or may not be linked to a common source or exposure.

The term "excess DNA" refers the amount of DNA use for restriction digest and will vary from about 5 ug to about 30 ug.

The term "chromatographic means" refers to any method of chromatographically separating nucleic acid fragments. Typical method include gel electrophoresis, high pressure liquid chromatography (HPLC), flow cytometry and membrane separation.

The term "banding pattern" means a pattern of DNA fragments distinctly separated on a gel or other chromatographic media.

The term "genetic fingerprint" means an array of DNA or RNA fragments or DNA or RNA nucleotide sequence from an organism which is sufficient to distinguish it from another organism of the same taxonomic classification at the level of genus, species, subspecies, serovar or strain designation.

The term "microbial" means bacteria, fungi, and parasites.

The term "epidemiologically linked" means independently isolated strains (isolated from a group of patients who have been exposed to the same vehicle of infection) which belong to the same species and are of clonal origin. In non-medical setting the term would indicate a group of isolated microbes which are of clonal origin.

The term "epidemiologically unlinked" means a group of isolates which are not of clonal origin.

The term "restriction endonuclease" or "restriction enzyme" will be abbreviated as RE and will mean an enzyme which binds and cuts within a specific nucleotide sequence within double-stranded DNA.

The term "4 base cutter" means a restriction endonuclease which recognizes and requires a specific DNA sequence of four nucleotides for site-specific digestion of double-stranded DNA.

The term "6 base cutter" means a restriction endonuclease which recognizes and requires a specific DNA sequence of six nucleotides for site-specific digestion of double-stranded DNA.

The term "genetically related population" refers to any grouping of organisms possessing multiple or single phenotypic characteristics of sufficient similarity to allow said organisms to be classified as a single genus, species, or subspecies.

The term "index of diversity" means the number of discrete subtypes identified by the number of isolates analyzed by a specific subtyping method.

"Nucleic acid" refers to a molecule which can be single-stranded or double-stranded, comprising monomers (nucleotides) containing a sugar, phosphate and either a purine or pyrimidine. In bacteria, lower eukaryotes, and in higher animals and plants, "deoxyribonucleic acid" (DNA) refers to the genetic material while "ribonucleic acid" (RNA) is involved in the translation of the information from DNA into proteins.

The present invention describes a method for the identification of genetic subtypes within a given related genetic population. The method utilizes the variation seen in a DNA banding pattern when genomic or chromosomal DNA is restricted with frequent base cutters. Only fragments between 0.1 and 18 kb are analyzed.

The present invention and its variations can be used for a myriad of applications. It may be used by plant or animal breeders to correctly identify their subjects, or it may be used by clinical and microbiological laboratories to identify bacteria, parasites or fungi present in any medium, including in eukaryotic cells. In this latter use, the method is preferred to the standard microbiological assays, since it does not require isolation and growth of the microbes. In vitro growth and characterization is now either impossible for some microorganisms such as *Mycobacterium leprae* (agent of leprosy), impossible on standard media for some microorganisms such as the obligate intracellular bacteria (e.g., *Rickettsia, Chlamydia,* etc.), or highly dangerous (e.g., *B. anthracis* (agent of anthrax). The present method depends on the isolation of nucleic acid and avoids these problems since it avoids conventional bacterial isolation and characterization.

The method is expected to characterize microorganisms that have not yet been conventionally described. In addition, the present method allows distinguishing different strains of species, and this can be useful, for example, for epidemiological typing in bacteriology. The method can be used by forensic laboratories to correctly and unambiguously identify plant or animal tissues in criminal investigations. It can also be used by entomologists to quickly identify insect species, when ascertaining the nature of crop infestations.

### Organisms Within A Genetically Related Population

The present method is useful for the determination of genetic subtypes within a specific genetically related population. That population may be represented by either prokaryotes or eukaryotes where the organisms are classified as a single genus, species, or subspecies. When identifying a prokaryotic organism, the DNA to be endonuclease-digested is that present in the cells or in the non-compartmentalized chromosomes. When identifying a eukaryotic organism nuclear DNA will be used. Preferred eukaryotic organisms that are amenable to analysis by the present method are microbial, and although may exist in an organized from such as are found in various fungal species, are generally unicellular.

Although any eukaryotic or prokaryotic organism is suitable for analysis by the instant method, the method is particularly suitable when used to analyze genera commonly associated with pathogenicity in human, animals and plants. Those genera include but are not limited to *Escherichia, Salmonella, Shigella, Vibrio, Listeria, Staphylococcus, Streptococcus, Enterococcus, Klebsiella, Neisseria, Pseudomonas* and *Campylobacter.*

### Isolation of DNA

The present method begins with the extraction and isolation of DNA from the organism to be analyzed. Although methods of DNA extraction from both prokaryotic and eukaryotic cells is well known in the art (see for example Sambrook, J. et al., Molecular Cloning: A Laboratory Manual, Second Edition, Cold Spring Harbor Laboratory Press, Cold Spring Harbor, NY (1989)), the methodology for each is slightly different.

For prokaryotic cells, an unknown bacterium present in any medium, such as an industrial fermentation suspension, agar medium, plant or animal tissue or sample or the like, is treated under well known conditions designed to extract high molecular weight DNA. For example, cells of the unknown organism can be suspended in extraction buffer, lysozyme added, and the suspension incubated. Cell disruption can be accelerated by addition of detergents, and/or by increase in temperature. Protease digestion followed by phenol/chloroform extraction and ethanol precipitation can be used to finalize the extraction of DNA. An alternative method of extraction, which is much faster than phenol/chloroform extraction, is rapid isolation of DNA using ethanol precipitation. This method is preferably used to isolate DNA directly from colonies or small, liquid cultures. The method is described in Davis, R. W. et al: "A Manual for Genetic Engineering, Advanced Bacterial Genetics", (hereinafter "Davis") Cold Spring Harbor Laboratory, Cold Spring, Harbor, NY, 1980, pp. 120-121.

Where the unknown organism is eukaryotic, an alternate methodology is used (see for example Drohan, W. et al, *Biochem*. *Biophys*. *Acta* 521 (1978) 1-15, herein incorporated by reference). Because organelle DNA is small and circular, spooling techniques serve to separate the non-circular nuclear DNA from the circular, organelle-derived DNA. As a corollary, the non-spooled material contains the organelle-derived DNA which can separately be isolated by density gradient centrifugation. Alternatively mitochondria (or chloroplasts) are separated from a mixture of disrupted cells; the purified mitochondrial (or chloroplast) fraction is used for the preparation of organelle-derived DNA while the purified nuclear fraction is used to prepare nuclear DNA. (See for example Bonn L. and Gray, M. W., *Nucleic Acids Research* 8:319-335 (1980)).

In addition to the general method cited above DNA may be isolated from the organism to be analyzed using a variety commercial kits available from industrial sources such as Stratagene (La Jolla, CA) and Promega Corp. (Madison, WI).

### Restriction Enzymes

The choice of restriction enzymes in the present invention is essential to the operability of the invention. Most suitable are restriction enzymes that are either 4, 5 or 6 base cutters wherein 4 base cutters are preferred. Four base cutters should have a recognition site, on average, every 256 bases along the DNA strand. Cuts at this frequency generate the large number of fragments needed for the analysis. Combinations of enzymes may also be used in a single digest where the digestion will result in the generation of nucleic acid fragments in the range of 0.1 to 18 kb. Single enzymes or combinations of enzymes that generate fragments outside of this range are not useful.

Suitable restriction enzymes useful in the present invention include but are not limited to *AccI, AluI, AvaI, AvaII*, *BanII*, *BfaI, CfoI, DdeI*, *DpnI, HaeII, HaeIII, HhaI*, *HincII, HinfI*, HpaII, *MboI, MnII, MseI, NciI, NlaIV*, RsaI, *Sau3AI, ScrfI*, *TaqI,* and *ThaI*. Methods for performing restriction digests are common and well known in the art (see for example Sambrook, J. et al., *supra).*

Selection of the appropriate enzymes for any given organism is accomplished by a process of comparing the digestions of a battery of enzymes against panel of organisms of the same taxonomic classification, epidemiologic history, or biochemical and morphologic characterization. For example, to differentiate between different strains or subtypes of a particular organism, genomic DNA is isolated from a panel of epidemiologically linked isolates as well as from a panel of epidemiologically un-linked isolates of the same genus. The isolated DNA is restricted using a large number of restriction endonucleases comprised of 4, 5 and 6 base cutters. Each enzyme employed is used in a single digestion reaction. The number of enzymes tested may vary where a panel of about 20 different enzymes (primarily 4 base cutters with some 5 and 6 base-specific cutters) is recommended. The restriction patterns or banding patterns are analyzed to identify the enzymes that will give the lowest index of diversity from the linked panel of organisms, and the highest index of diversity from the unlinked panel. The index of diversity is determined by dividing the number of banding patterns over the number of organisms tested. A pattern is considered significant if all the bands generated are less than 18 kb and discrete bands can be identified. When comparing banding patterns, the presence of only one additional band is considered significant and will represent a distinct subtype. Similarly, a difference in band intensity may define a significant variation. For example, where the banding patterns are identical with respect to the number and size of the DNA fragments, but where one or more bands differ in intensity, the difference in band intensity will be considered evidence of a distinct subtype.

Enzymes chosen on the basis of these distinctions will then be used to subtype all organisms falling into a particular species or genus.

Although frequent cutting endonucleases are effective at generating significant banding patterns, it is also possible to use more than one infrequent cutting enzyme to achieve the same effect. For example, several 6 base cutters may be used in a double, or triple digest. Depending on the organism chosen the multiple digest will generate an banding pattern of equal efficacy to that generated by a frequent base cutter. When more than one enzyme is used in inter- or intrastrain differentiation studies, their results are combined; genetic identity or clonality may then be defined as groups of independent isolates which have identical banding patterns with all the enzymes used in the study.

### Chromatographic and Electrophoretic Methods of DNA separation

Digested DNA must be separated into a discrete banding pattern or fingerprint for subtyping to be effective. Comparison of the distinct genetic fingerprint of the organism under study with the genetic fingerprint of a specific genetic sub-type will then afford differentiation of genetic sub-types within a population of genetically related organisms.

Any method of separating DNA fragments is suitable in the present invention including HPLC, flow cytometry, membrane chromatography and gel electrophoresis. Methods of DNA separation by HPLC are common and well known in the art (see for example Wages et al., High Performance Liquid Chromatography*: Principal Methods Biotechnology* (1996), 351-379. Editor(s): Katz, Elena D. Publisher: Wiley, Chichester, UK.; Takenaka et al., *Curr*. *Innovations Mol. Biol*. (1995), Volume Date 1995, 2, 137-54). Similarly methods of DNA separation using membrane chromatography are possible, where the DNA fragments are labeled with suitable reporters (see for example, U.S. 5,310,650 and WO 9527081). Additionally, flow cytometry has been used for separation of fluorescently labeled restriction fragments (See for example Huang, et al., *Nuc*. *Acids. Res.,* 24, 4202, (1996)).

The separation method of choice in the present invention is gel electrophoresis. Isolated and restricted DNA is subjected to electrophoretic separation to develop a specific and unique banding pattern. Any method of electrophoresis that is amenable to the separation of nucleic acid fragments is suitable. For example, DNA fragments may be separated using gels comprised of agarose or polyacrylamide and may be non-denaturing or denaturing. The gel system may be as capillary gel electrophoresis, pulsed field gel electrophoresis, field inversion electrophoresis or 2 dimensional gel electrophoresis.

Methods for the electrophoretic separation of nucleic acids are well known in the art, see for example Suvorovet al., Adv. Exp. Med. Biol. (1997), 418(Streptococci and the Host), 979-981; Dovichi N., *Autom. Technol. Genome Charact*. (1997), 145-165. Editor(s): Beugelsdijk, Tony J. Publisher: Wiley, New York, NY; Caetano-Anolles, G., Fingerprinting Methods Based Arbitrarily Primed PCR (1997), 119-134. Editor(s): Micheli, Maria Rita; Bova, Rodolfo. Publisher: Springer, Berlin, Germany.

Preferred in the present method are agarose gels comprised of about 0.6% to about 2% agarose, where a concentration of about 1% agarose is most preferred. Gels are typically run under non-denaturing conditions and over a time period from about 4 hours to about 20 hours where a time of about 17 hours is preferred. Voltage potential across the gel may be varied to enhance fragment separation. Lower voltages of about 20v give less distinct patterns as oppose to voltages in the 70v to 100v ranges. Where standard horizontal electrophoretic methods are employed it is useful to resolve the banding pattern within specific area of the gel. Generally the best results are obtained when the distance between fragments with the greatest and lowest molecular weight within the data interpretation zone (starting at the fragment with the largest molecular weight and going down to 1-2 kb fragments) is in the 4 cm to 12 cm range. In a preferred embodiment it is useful to run fragments less than 2 kb off the gel to improve banding pattern resolution.

### DNA Binding Agents

The present method employs a DNA binding agent for the identification of DNA bands after gel electrophoresis. Particularly suited are DNA intercalating agents capable of binding to DNA and emitting a fluorescent signal. A variety of intercalating agents are known in the art such as propidium iodide (PI) and ethidium bromide (EB) [Sailer et al., *Cytometry* (1996), 25(2), 164-172] and the cyanine dyes such as Oxazole Yellow [EP 714986], TOTO (1,1'-(4,4,7,7-tetramethyl-4,7-diazaundecamethylene)-bis-4-[3-methyl-2,3-dihydro-(benzo-1,3-thiazole)-2-methylidene]-quinolinium tetraiodide), a homodimer of thiazole orange [Axton et al., *Mol*. *Cell. Probes* (1994), 8(3), 245-50] oxazole orange (YOYO)[ Srinivasan et al., *Appl*. *Theor. Electrophor.* (1993), 3(5), 235-9] SybrGreen™, available from Molecular Probes, Inc. (Eugene, OR, USA), and others included in U.S. Patent No. 5,563,037, hereby incorporated by reference. Preferred in the present method are ethidium bromide and propidium iodide, readily available from Sigma Chemical Company (St. Louis, MO).

The present invention is further defined in the following Examples. It should be understood that these Examples, while indicating preferred embodiments of the invention, are given by way of illustration only. From the above discussion and these Examples, one skilled in the art can ascertain the essential characteristics of this invention, and without departing from the spirit and scope thereof, can make various changes and modifications of the invention to adapt it to various usages and conditions.

### EXAMPLES

### GENERAL METHODS

Procedures for DNA restriction analysis and other protocols common in the art of molecular biology used in the following examples may be found in Sambrook, J. et al., Molecular Cloning: A Laboratory Manual, Second Edition, Cold Spring Harbor Laboratory Press, Cold Spring Harbor, NY (1989) and by T. J. Silhavy, M. L. Bennan, and L. W. Enquist, Experiments with Gene Fusions, Cold Spring Harbor Laboratory, Cold Spring Harbor, NY (1984) and by Ausubel, F. M. et al., Current Protocols in Molecular Biology, pub. by Greene Publishing Assoc. and Wiley-Interscience (1987).

Materials and methods suitable for the maintenance and growth of bacterial cultures are well known in the art. Techniques suitable for use in the following examples may be found in Manual of Methods for General Bacteriology (Phillipp Gerhardt, R. G. E. Murray, Ralph N. Costilow, Eugene W. Nester, Willis A. Wood, Noel R. Krieg and G. Briggs Phillips, eds), American Society for Microbiology, Washington, DC. (1994) or in the work of Thomas D. Brock (in Biotechnology: A Textbook of Industrial Microbiology, Second Edition (1989) Sinauer Associates, Inc., Sunderland, MA). All reagents and materials used for the growth and maintenance of bacterial cells were obtained from Aldrich Chemicals (Milwaukee, WI), DIFCO Laboratories (Detroit, MI), GIBCO/BRL (Gaithersburg, MD), or Sigma Chemical Company (St. Louis, MO) unless otherwise specified.

Restriction enzymes used in the following examples were obtained from American Allied Biochemicals (Aurora, CO), New England Biolabs (Beverly, MA) and Boehringer-Mannheim (Indianapolis, IN).

The meaning of abbreviations is as follows: "h" means hour(s), "min" means minute(s), "sec" means second(s), "d" means day(s), "uL" means microliters, "mL" means milliliters, and "L" means liters.

### Bacterial strains

*Escherichia coli O157:H7* strains were obtained from Washington State Health Department, King County laboratories, and Center for Disease Control and Prevention and grown on tryptic soy agar (Difco Laboratories).

### Growth Conditions

All strains were grown overnight on tryptic soy agar. Confluent bacterial growth on agar plates was scraped with several sweeps of a sterile flat-headed toothpick and suspended in 0.8 mL of 50 mM Tris (pH 8.0) containing 50 mM EDTA. Forty-five uL of 20% sodium dodecyl sulfate (SDS) and 10 uL of Proteinase K (20 mg/mL; Boehringer-Mannheim, Indianapolis, IN) was added to all suspensions. The suspensions were incubated at 50°C for 1 h.

### Isolation of DNA

### DNA Isolation from Gram Negative Bacteria:

Confluent bacterial growth on agar plates was scraped with several sweeps of a sterile flat-headed toothpick and suspended in 0.8 mL of 50 mM Tris, 50 mM EDTA (pH 8.0). Forty-five uL of 20% sodium dodecyl sulfate (SDS) and 10 uL of proteinase K (20 mg/mL ; Pharmacia, Piscataway, N.J.) was added to all suspensions. The suspensions were incubated at 50°C for 1 h. Phenol-chloroform and chloroform extractions are performed on the DNA suspensions. The DNA is precipitated with the addition of 2.5 times the volume of absolute ethanol, spooled with a glass capillary pipette and suspended in 100-200 uL sterile dH₂O.

### DNA Isolation from Gram Positive Bacteria:

Confluent bacterial growth on agar plates was scraped with several sweeps of a sterile flat-headed toothpick and suspended in 0.8 mL of 50 mM Tris, 50 mM EDTA (pH 8.0), plus 10 mg/mL lysozyme and incubated for 1 h, at 37°C. Fifty uL of 5 mg/mL solution of Lysostaphin (Sigma, St. Louis, MO) was added to each tube and the mixture was incubated for 1 h, at 37°C. Forty-five uL of 20% sodium dodecyl sulfate (SDS) and 10 uL of proteinase K (20 mg/mL; Pharmacia, Piscataway, NJ) was added to all suspensions. The suspensions were incubated at 50°C for 1 h. Phenol-chloroform and chloroform extractions are performed on the DNA suspensions. The DNA is then precipitated with the addition of 2.5 times the volume of absolute ethanol, spooled with a glass capillary pipette and suspended in 100-200 uL sterile dH₂O.

### Restriction Digests:

Ten to twenty microliters from each DNA preparations (1 ug/uL) was digested with 10-20 units of appropriate restriction enzymes in a 30 uL reaction volume according to the manufacturer's instructions. All DNA preparations were digested for a minimum of 2 h at 37°C (unless other temperatures were indicated by the manufacturer).

### Electrophoresis

Following digestion, DNA fragments were electrophoretically separated on 15x20 cm agarose gels in 1X Tris-borate EDTA. Unless otherwise specified the gels were 1% agarose. Unless otherwise specified the gels were run for 17 h at 70 v. Lambda-Hind III and 1KB ladder were used as the molecular size markers. Gels were stained with ethidium bromide for 20 min, destained with deionized water for 1 h, and visualized.

### EXAMPLE 1

### SUBTYPING OF E. COLI 0157

Example 1 demonstrates the process for the selection of appropriate restriction enzymes for the subtyping of a particular organism and the subsequent determination of a genetic subtype.

Two panels of *E. coli 0157* isolates with known epidemiological history were assembled. In order to identify appropriate restriction enzymes, groups of *E*. *coli* O157:H7 strains were analyzed with various enzymes.

Genomic DNA was isolated and extracted from the members of each panel according to the protocol described in the GENERAL METHODS. Extracted DNA was then restricted with a variety of enzymes listed below:
*Alu1, Asc1, Ava1, AvaII, BamH1, BfaI, BstuI, BspI, DdeI, DpnI, EcoR1, HincII, HindIII, HaeIII, HinF1, HhaI, Mnl1, Msp1, Nci1, nlaIII, NlaIV, PvuII, Rsa1*, *Sau3AI, Scrf1, Taq1*.

Restriction digested proceeded according to the method described in the GENERAL METHODS, and the restriction fragments were resolved by gel electrophoresis, with the exception that the voltage was varied between 22-34 volts. The resulting banding patterns were analyzed to identify the restriction enzymes which could potentially generate epidemiologically relevant data. Enzymes generating DNA fragments no larger than 18 kb, where the majority of bands are less than 12 kb are selected for further use.

Upon further examination, of the 14 enzymes, several were excluded and due to lack of sensitivity in inter-strain differentiation of *E*. *coli O157:H7*, or the nature of their banding patterns. For example *AvaII* was excluded because it generated a very compressed banding banding pattern in the 18 kb size range. *Hinf1* and *NlaIII* were excluded because almost all the bands were below 4 kb which resulted in a very compressed and indecipherable banding pattern.

Several of the enzymes were selected for further testing results are shown in Figures 2a-i. From this smaller set of enzymes, *HaeIII* and *Sau3AI* were selected as the primary and secondary enzymes to be used for *E*. *coli O157:H7* subtyping.

DNA from a panel of unlinked isolates were restricted with *HaeIII, Sau3AI, Nci1* and *Alu1* and the fragments resolved by gel electrophoresis (Figure 2i). As can been seen by the data, *Sau3AI* and *Hae III* were clearly superior to *Nci1* and *Alu1* in inter-strain differentiation of *E. coli O157:H7*. It is also clear that *Nci1* and *Alu1* may be used in situations where more genomic characterization is needed. Figures 3a-b demonstrate the use of *Sau3AI* and *Hae3A* on a panel of epidemiologically linked and unlinked isolates. Lanes 1, 3, and 4 in each figure represent epidemiologically unlinked isolates, and lanes 2, 5, 6, 7, and 8 represent epidemiologically linked strains. Each enzyme separates the unlinked isolates from each other and from the linked ones. Each of the two enzymes gave identical banding patterns for the members of the linked panel.

### EXAMPLE 2

### EFFECT OF RESTRICTION FRAGMENT SIZE ON PATTERN SPECIFICITY

Example 2 compares use of restriction enzymes which produce banding patterns below 18 kb (frequent cutters) over enzymes with less frequent recognition sites (generally 6 base cutters) in the present method.

*E. coli 0157* cells from three different strains were prepared as described in Example 1. Genomic DNA was extracted and separated into four pools. One pool was restricted with the 4 base cutter *Sau3AI* and the other pools with the 6 base cutters *EcoR1*, *Hind III* and *PvuII*.

As can be seen by the data, the banding patterns generated by the 6 base cutters are unable to generate a significant banding fingerprint. In contrast, the banding pattern generated by *Sau3AI* is very informative and easy to interpret. The three strain which had no epidemiological connection show banding patterns which are different from each other.

### EXAMPLE 3

### BACTERIAL SUBTYPING BY DOUBLE DIGEST WITH RARE CUTTING ENDONUCLEASES

Example 3 illustrates that rare cutting restriction endonucleases, when used in combination, have the ability to produce a significant banding fingerprint, similar to that achieved by using frequent cutting enzymes.

*E*. *coli 0157* cells were prepared as described in Example 1. Genomic DNA was extracted and separated into 3 pools. One pool was restricted with *Sma1* and *HincII*, the second pool was cut with *Pst1* and *HindIII*, and the third was restricted using *HindIII* and *EcoRV*. As it is evident from Figure 5 combination of *HincII* and *Sma1* resulted in generating fragments less than 18 kb in a significant banding fingerprint.

### EXAMPLE 4

### EFFECT OF DNA CONCENTRATION ON RESTRICTION PATTERN

Example 4 demonstrates the effect of starting concentration of DNA on the resolution of restricted fragments by gel electrophoresis.

*E. coli 0157* cells were prepared as described in Example 1. Genomic DNA was extracted and aliquoted for restriction digest. 1 ug, 2 ug, 4 ug, 8 ug, 16 ug, and 20 ug of DNA were restricted in separate reactions using *HaeIII* and *Sau3AI*. The resulting banding patterns were resolved by gel electrophoresis according to the protocol in the GENERAL METHODS.

Banding patterns are seen clearly at 16-20 ug range. In contrast 1-2 ug of DNA is sufficient to obtain a banding pattern with 6 base cutters; however, as has been demonstrated, such cutters used alone are not effective in the present method.

### EXAMPLE 5

### EFFECT OF AGAROSE VARIATION ON THE RESOLUTION OF BANDING PATTERNS

DNA was prepared as described above from 3 different panels of *E*. *coli O157:H* strains. DNA from two of the panels were restricted with *NciI* and *Sau3AI* and applied to gels with varying concentrations of agarose. All gels were run for 17 hr and at 45 volts. Figures 7a and b illustrate the difference in separation when the agarose concentration is varied between 1% and 2%. The more concentrated agarose produces more compressed banding patterns which will limit the utility of the method.

DNA from the third panel was separated into 3 pools; each pool was digested using *Sau3AI*. The three pools were applied to three different agarose gels with agarose concentrations of 0.7%, 0.8% and 1% (Figures 8a, 8b, and 8c, respectively). Electrophoresis was conducted for 17 h, at 45 volts. As is evident from the data, the resolution improves with lower concentrations of agarose. However, the smaller bands (around 3-4 kb and lower) become noticeably diffused.

### EXAMPLE 6

### DEMONSTRATION OF METHOD ON VARIETY OF BACTERIA

Example 6 demonstrates that the present method of bacterial subtyping is applicable to a diverse number of gram positive and gram negative bacterial genera.

All strains were grown according to the protocols in the GENERAL METHODS and DNA was extracted and prepared for restriction analysis. Genomic DNA was extracted from *Listeria, Salmonella, Shigella, E. coli, Klebsiela, Pseudomonas, Vibrio, Staphylococcus,* and *Neisseria*.

### Salmonella and Shigella

To identify the enzymes that produce banding patterns in the desired size range (less than 18 kb), a panel of three strains was assembled, consisting of one *E. coli 0157*:*H* (#1706), one *Shigella sonnei* (#14), and one *Salmonella bovismorbificans* (#66). Isolates were grown, and DNA was isolated as described in the general methods section. DNA was restricted with *AviI, Bstul, Dpnl, HhaI, MspI, NlaIII, NlaIV, RsaI,* and *ScrFI*. A separate panel of *S*. *sonnei* isolates were tested by *HaeIII*, *NciI* and *Sau3AI*.

Figures 10a and 10b show the use of *Sau3AI* and *HaeIII* on a panel of *S. sonnei* isolates of known epidemiological history (containing linked and unlinked cases). *Sau3AI* alone divided the isolates into appropriate epidemiological groups. Although *HaeIII* showed less sensitivity, it demonstrated good differentiative power, qualifying it as one of the secondary enzymes for *Shigella.* Several of the other enzymes including *NciI* were also deemed to be useful in the study of *Shigella spp.*

A panel of 11 *Salmonella bovismorbificans* isolates of known epidemiological history were assembled. The isolates were grown, and DNA was purified as described above. The DNA preps were restricted with *HaeIII*, *AluI*, *NciI*, and *Sau3AI*. Both *Sau3AI* and *NciI* showed that lanes 5-13 had identical banding patterns, and lanes 3 and 4 each had unique patterns. The data confirmed the epidemiological data which showed that strains 5-13 were from a single cluster and strains 3 and 4 were unlinked isolates. *Alul* could not separate the unlinked isolates from each other and from the linked isolates. *HaeIII* separated the unlinked from the linked isolates but it could not separate them from each other.

### Neisseria

A panel of *Neisseria meningitidis* isolates was grown, DNA was isolated (as described in the GENERAL METHODS), and tested with *AscI, BfaI, DpnII, HaeIII*, *MnII,* and *Sau3AI* (*DpnII* and *Sau3AI* have identical recognition sequences). The results indicate that *HaeIII*, and *Sau3AI*/*DpnII* were equally useful for *Neisseria* species.

### Pseudomonas aeruginosa, Klebsiella pneumoniae, and Vibrio parahaemolyticus

On the basis of the previous results for other gram negatives (*E*. *coli, Salmonella bovismorbificans and Shigella sonnei*), *Sau3AI*, *HaeIII*, and *NciI* were chosen to be tested on *Pseudomonas aeruginosa*. *NciI* was selected as one of the primary enzymes for *Pseudomonas aeruginosa*. The enzyme separated the linked and the unlinked isolates from each other and the subtyping data 100% matched the epidemiological data.

As can be seen by the data, both enzymes divided the group according to the epidemiological linkages.

Utility of the method in interstrain differentiation of *Vibrio parahaemolyticus*. A panel of *V*. *parahaemolyticus* isolates with known epidemiological history was analyzed with *Sau3AI* and *HaeIII*. Both enzymes proved to be very powerful in differentiation of the *V*. *parahaemolyticus* isolates. Combination of the results of the two enzymes showed the true linkages of the isolates.

### Enterococci spp.

On the basis of the results obtained with other gram positives *(Staphylococcus aureus* and *Listeria monocytogenes* - see below), a panel of *Enterococci* isolates with known epidemiological history was obtained and tested with *HaeIII*, and *HhaI*. *HaeIII* and *HhaI* resulted in epidemiologically relevant data. The electrophoresis conditions were revised to employ a 6% agarose gel for analysis of *Enterococci* with *HaeIII* and *HhaI*.

### Staphylococcus aureus

Two panels of *Staphylococcus aureus* strains of known epidemiological history were assembled. Cells were grown and DNA was isolated as described above. One member of the panel was tested with a panel of restriction enzymes. The data is shown in Figure 15a where Lane 1, is 1 kb ladder, lanes 2-14 are *Staphylococcus aureus* DNA cut with *Alul, AscI, AvaI*, *AvaII*, *Bfal, Bstul, DdeI*, *DpnII, MnII*, *Msel, MspI, Rsal,* and *ScrFI*, respectively. *Sau3AI, HaeIII, HhaI*, *NciI*, and *NlaIV* were also tested with a *Staphylococcus aureus* isolate.

Several of the enzymes used above generated the banding of appropriate size range (starting at or below 18 kb). The results obtained from each one of the enzymes validated the epidemiological linkages which were available for the panel. *DdeI, MnlI,* and *HhaI* were used on the second panel of *Staphylococcus* isolates.

### Listeria monocytogenes

A panel of *Listeria* strains of known epidemiological history were assembled, cells were grown, and DNA was extracted as described above. To determine the appropriate enzymes, *Listeria monocytogenes* DNA was restricted with a panel of enzymes. Lane 1, 1KB ladder, lanes 2-14 are *Listeria monocytogenes* DNA restricted with *AscI, AvaI, Avall, Bfal, BstuI, DdeI, DpnI, MnlI, MseI, MspI*, *NlaIV, RsaI,* and *ScrFI*, respectively.

*Listeria monocytogenes* DNA was separately tested with *HaeIII, AluI, Sau3AI, HhaI, DpnII*, and *NciI*. Several of the enzymes generated the desired banding size range (starting at or below 18KB). Based on these tests *DpnII* was selected as an appropriate enzyme for subtyping.

## Claims

1. A method for the differentiation of genetic sub-types within a population of genetically related organisms comprising the steps of:
(i) isolating genomic DNA from an organism to be analyzed;
(ii) restricting genomic DNA from said organism with at least one restriction endonuclease to yield restricted DNA wherein all fragments are less than 18kb;
(iii) resolving said restricted DNA by chromatographic means capable of separating fragments in the 0.1 to 18 kb range to generate a distinct genetic fingerprint;
(iv) comparing said distinct genetic fingerprint of step (iii) with the genetic fingerprint of a specific genetic sub-type wherein differences in said genetic fingerprints afford differentiation of genetic sub-types within a population of genetically related organisms.

2. A method according to Claim 1 wherein said organism is selected from the group consisting of prokaryotes and microbial eukaryotes.

3. A method according to Claim 2 wherein said organism is selected from the group consisting of gram positive bacteria and gram negative bacteria.

4. A method according to Claim 3 wherein said organism is selected from the group consisting of *Escherichia, Salmonella*, *Shigella*, *Vibrio*, *Listeria*, *Staphylococcus*, *Streptococcus, Enterococci, Klebsiela, Nicericia Pseudomonas* and *Campylobacter*.

5. A method according to Claim 4 wherein said organism is selected from the group consisting of *E. coli 0157* and *Salmonella.*

6. A method according to Claim 1 wherein said chromatographic means of step (iii) is selected from the group consisting of gel electrophoresis, HPLC, flow cytometry, and membrane chromatography.

7. A method according to Claim 6 wherein said method of gel electrophoresis is selected from the group consisting of capillary gel electrophoresis, pulsed field gel electrophoresis, 2 dimensional gel electrophoresis, and field inversion electrophoresis.

8. A method according to Claim 7 wherein said gel is an agarose gel having a concentration of from about 0.6% to about 2% agarose.

9. A method according to Claim 7 wherein said gel is polyacrylamide.

10. A method according to Claim 7 wherein said restricted DNA is stained with a DNA binding dye selected from the group consisting of ethidium bromide, propidium iodide, and fluorescent cyanine dyes.

11. A method according to Claim 1 wherein said at least one restriction enzyme is selected from the group consisting of *AccI*, *AluI*, *AvaI*, *AvaII*, *BanII*, *BfaI, CfoI*, *DdeI*, *DpnI*, *HaeII*, *HaeIII*, *HhaI*, *HincII*, *HinfI*, HpaII, *MboI*, *MnII*, *MseI*, *NciI*, *NlaIV*, *RsaI*, *Sau3AI*, *ScrfI*, *TaqI*, and *ThaI*.

12. A method according to Claim 1 wherein said at least one restriction enzyme is a 4 base cutter.

13. A method according to Claim 1 wherein said at least one restriction enzyme is a combination of at least two 6 base cutters.

14. A method according to Claim 1 wherein said genomic DNA is from about 5 ug to about 30 ug.

15. A method according to Claim 1 wherein said resolving of restricted DNA at step (iii) results in the elimination of all DNA fragments less than 2 kb.

16. A method for the interstrain differentiation of bacterial isolates comprising the steps of:
(i) isolating genomic DNA from a) a panel of epidemiologically linked bacterial isolates and b) a panel of epidemiologically un-linked bacterial isolates wherein said linked and un-linked isolates are members of the same genus;
(ii) restricting genomic DNA from the linked and un-linked panel of isolates with a panel of restriction enzymes selected from the group consisting of *AccI*, *AluI*, *AvaI*, *AvaII*, *BanII*, *BfaI, CfoI*, *DdeI*, *DpnI, HaeII*, *HaeIII*, *HhaI, HincII*, *HinfI*, *HpaII*, *MboI*, *MnII*, *MseI, NciI*, *NlaIV*, *RsaI*, *Sau3AI*, *ScrfI*, *TaqI*, and *ThaI*;
(iii) resolving the restricted DNA of step (ii) by gel electrophoresis to generate a banding pattern for each enzyme;
(iv) selecting at least one enzyme from the panel of enzymes of step (ii) that a) restricts all of the genomic DNA to fragments of less than 18 kb, b) generates the lowest index of diversity from the linked panel and c) generates the highest index of diversity from the unlinked panel;
(v) restricting the isolated DNA from the linked and un-linked panels of bacterial isolates of step (i) with the at least one enzyme of step (iv);
(vi) resolving said restricted DNA of step (v) by gel electrophoretic means capable of separating fragments in the 0.1 to 18 kb range to generate a distinct genetic fingerprint for said linked and unlinked isolates; and
(vii) comparing said distinct genetic fingerprints of step (vi) wherein differences in said genetic fingerprints affords interstrain differentiation of said bacterial isolates.

17. A method according to Claim 16 wherein said method of gel electrophoresis is selected from the group consisting of, capillary gel electrophoresis, pulsed field gel electrophoresis, 2 dimensional gel electrophoresis, inversional electrophoresis and vertical electrophoresis.

18. A method according to Claim 16 wherein said gel is an agarose having a concentration of from about 0.6% to about 2% agarose.

19. A method according to Claim 16 wherein said gel is polyacrylamide.

20. A method according to Claim 16 wherein said restricted DNA is stained with a DNA binding dye selected from the group consisting of ethidium bromide, propidium iodide, and fluorescent cyanine dyes.

21. A method according to Claim 16 wherein said at least one restriction enzyme is a 4 base cutter.

22. A method according to Claim 16 wherein said at least one restriction enzyme is a combination of at least one 4 base cutter and at least one 6 base cutter.

23. A method according to Claim 16 wherein said genomic DNA is from about 5 ug to about 30 ug.

24. A method according to Claim 16 wherein said resolving of restricted DNA at step (iii) results in the elimination of all DNA fragments less than 2 kb.

25. A method according to Claim 16 wherein said resolving of restricted DNA at step (iii) results in separation of DNA fragments over an distance of about 4 cm to about 12 cm of gel.

26. A method according to Claim 16 wherein said bacterial isolate is selected from the group consisting of *Escherichia, Salmonella, Shigella, Vibrio, Listeria, Staphylococcus, Streptococcus, Enterococci, Klebsiela, Nicericia Pseudomonas* and *Campylobacter*.

27. A method according to Claim 16 wherein said bacterial isolate is selected from the group consisting of *E*. *coli 0157* and *Salmonella*.

## Patentansprüche

1. Verfahren zur Unterscheidung genetischer Subtypen innerhalb einer Population genetisch verwandter Organismen, folgende Schritte aufweisend:
(i) Isolieren genomischer DNA von einem zu analysierenden Organismus;
(ii) Restriktionsspalten genomischer DNA dieses Organismus mit mindestens einer Restriktionsendonuclease zur Erzielung restriktionsgespaltener DNA, wobei alle Fragmente kleiner als 18 kb sind;
(iii) Auftrennen der restriktionsgespaltenen DNA durch chromatografische Mittel, die in der Lage sind, Fragmente in dem Bereich von 0,1 bis 18 kb zu trennen, um einen eindeutigen genetischen Fingerabdruck zu erzeugen;
(iv) Vergleichen des eindeutigen genetischen Fingerabdrucks aus Schritt (iii) mit dem genetischen Fingerabdruck eines spezifischen genetischen Subtyps, wobei Unterschiede der genetischen Fingerabdrücke die Unterscheidung genetischer Subtypen innerhalb einer Population genetisch verwandter Organismen erlauben.

2. Verfahren nach Anspruch 1, bei dem der Organismus ausgewählt ist aus der Gruppe, die aus Prokaryoten und Mikroben-Eukaryoten besteht.

3. Verfahren nach Anspruch 2, bei dem der Organismus ausgewählt ist aus der Gruppe, die aus grampositiven Bakterien und gramnegativen Bakterien besteht.

4. Verfahren nach Anspruch 3, bei der der Organismus ausgewählt ist aus der Gruppe, die besteht aus Escherichia, Salmonella, Shigella, Vibrio, Listeria, Staphylococcus, Streptococcus, Enterococci, Klebsiela, Nicericia Pseudomonas und Campylobacter.

5. Verfahren nach Anspruch 4, bei dem der Organismus ausgewählt ist aus der Gruppe, die aus E. coli 0157 und Salmonella besteht.

6. Verfahren nach Anspruch 1, bei dem die chromatografischen Mittel aus Schritt (iii) ausgewählt sind aus der Gruppe, die besteht aus Gelelektrophorese, HPLC, Durchflußcytometrie und Membranchromatografie.

7. Verfahren nach Anspruch 6, bei dem das Verfahren der Gelelektrophorese ausgewählt ist aus der Gruppe, die besteht aus Kapillargelelektrophorese, Pulsfeldgelelektrophorese, zweidimensionaler Gelelektrophorese und Feldumkehrelektrophorese.

8. Verfahren nach Anspruch 7, bei dem das Gel ein Agarosegel mit einer Konzentration von etwa 0,6 % bis etwa 2 % Agarose ist.

9. Verfahren nach Anspruch 7, bei dem das Gel Polyacrylamid ist.

10. Verfahren nach Anspruch 7, bei dem die restriktionsgespaltene DNA mit einem DNA-Bindungsfarbstoff, der ausgewählt ist aus der Gruppe, die aus Ethidiumbromid, Propidiumiodid und fluoreszierenden Cyaninfarbstoffen besteht, gefärbt wird.

11. Verfahren nach Anspruch 1, bei dem das mindestens eine Restriktionsenzym ausgewählt wird aus der Gruppe, die besteht aus AccI, AluI, AvaI, AvaII, BanII, BfaI, CfoI, DdeI, DpnI, HaeII, HaeIII, HhaI, HincII, HinfI, HpaII, MboI, MnII, MseI, NciI, NlaIV, RsaI, Sau3AI, ScrfI, TaqI und ThaI.

12. Verfahren nach Anspruch 1, bei dem das mindestens eine Restriktionsenzym ein 4-Basen-Schneider ist.

13. Verfahren nach Anspruch 1, bei dem das mindestens eine Restriktionsenzym eine Kombination von mindestens zwei 6-Basen-Schneidern ist.

14. Verfahren nach Anspruch 1, bei dem die genomische DNA von etwa 5 ug bis etwa 30 ug hat.

15. Verfahren nach Anspruch 1, bei dem das Auftrennen restriktionsgespaltener DNA in Schritt (iii) zur Beseitigung aller DNA-Fragmente von weniger als 2 kb führt.

16. Verfahren zur Unterscheidung zwischen Stämmen von Bakterienisolaten, folgende Schritte aufweisend:
(i) Isolieren genomischer DNA aus a) einer Reihe epidemiologisch verknüpfter Bakterienisolate und b) einer Reihe epidemiologisch nicht verknüpfter Bakterienisolate, wobei die verknüpften und die nicht verknüpften Isolate Mitglieder derselben Gattung sind;
(ii) Restriktionsspalten genomischer DNA aus der verknüpften und der nicht verknüpften Reihe von Isolaten mit einer Reihe von Restriktionsenzymen, die ausgewählt sind aus der Gruppe, die besteht aus AccI, AluI, AvaI, AvaII, BanII, BfaI, CfoI, DdeI, DpnI, HaeII, HaeIII, HhaI, HincII, HinfI, HpaII, MboI, MnII, MseI, NciI, NlaIV, RsaI, Sau3AI, ScrfI, TaqI und ThaI;
(iii) Auftrennen der restriktionsgespaltenen DNA aus Schritt (ii) durch Gelelektrophorese zur Erzeugung eines Bandingmusters für jedes Enzym;
(iv) Auswählen mindestens eines Enzyms aus der Reihe von Enzymen von Schritt (ii), das a) die gesamte genomische DNA in Fragmente von weniger als 18 kb restriktionsspaltet, b) von der verknüpften Reihe den niedrigsten Diversitätsindex erzeugt und c) von der nicht verknüpften Reihe den höchsten Diversitätsindex erzeugt;
(v) Restriktionsspalten der von den verknüpften und den nicht verknüpften Reihen von Bakterienisolaten isolierten DNA aus Schritt (i) mit dem mindestens einen Enzym aus Schritt (iv);
(vi) Auftrennen der restriktionsgespaltenen DNA aus Schritt (v) durch gelelektrophoretische Mittel, die in der Lage sind, Fragmente in dem Bereich von 0,1 bis 18 kb zu trennen, um einen eindeutigen genetischen Fingerabdruck für die verknüpften und die nicht verknüpften Isolate zu erzeugen; und
(vii) Vergleichen der eindeutigen genetischen Fingerabdrücke aus Schritt (vi), wobei Unterschiede der genetischen Fingerabdrücke eine Unterscheidung zwischen Stämmen der Bakterienisolate erlauben.

17. Verfahren nach Anspruch 16, bei dem das Verfahren der Gelelektrophorese ausgewählt ist aus der Gruppe, die besteht aus Kapillargelelektrophorese, Pulsfeldgelelektrophorese, 2-dimensionaler Gelelektrophorese, Inversionselektrophorese und vertikaler Elektrophorese.

18. Verfahren nach Anspruch 16, bei dem das Gel ein Agarosegel mit einer Konzentration von etwa 0,6 % bis etwa 2 % Agarose ist.

19. Verfahren nach Anspruch 16, bei dem das Gel Polyacrylamid ist.

20. Verfahren nach Anspruch 16, bei dem die restriktionsgespaltene DNA mit einem DNA-Bindungsfarbstoff, der ausgewählt ist aus der Gruppe, die besteht aus Ethidiumbromid, Propidiumiodid und fluoreszierenden Cyaninfarbstoffen, gefärbt wird.

21. Verfahren nach Anspruch 16, bei dem das mindestens eine Restriktionsenzym ein 4-Basen-Schneider ist.

22. Verfahren nach Anspruch 16, bei dem das mindestens eine Restriktionsenzym eine Kombination von mindestens einem 4-Basen-Schneider und mindestens einem 6-Basen-Schneider ist.

23. Verfahren nach Anspruch 16, bei dem die genomische DNA von etwa 5 ug bis 30 ug hat.

24. Verfahren nach Anspruch 16, bei dem das Auftrennen restriktionsgespaltener DNA in Schritt (iii) zur Beseitigung aller DNA-Fragmente von weniger als 2 kb führt.

25. Verfahren nach Anspruch 16, bei dem das Auftrennen restriktionsgespaltener DNA in Schritt (iii) zur Trennung von DNA-Fragmenten über eine Entfernung von etwa 4 cm bis etwa 12 cm des Gels führt.

26. Verfahren nach Anspruch 16, bei dem das Bakterienisolat ausgewählt wird aus der Gruppe, die besteht aus Escherichia, Salmonella, Shigella, Vibrio, Listeria, Staphylococcus, Streptococcus, Enterococci, Klebsiela, Nicericia Pseudomonas und Campylobacter.

27. Verfahren nach Anspruch 16, bei dem das Bakterienisolat ausgewählt wird aus der Gruppe, die besteht aus E. Coli 0157 und Salmonella.

## Revendications

1. Procédé pour la différenciation de sous-types génétiques au sein d'une population d'organismes génétiquement apparentés comprenant les étapes de :
(i) isoler l'ADN génomique d'un organisme à analyser ;
(ii) effectuer une restriction de l'ADN génomique dudit organisme, avec au moins une endonucléase de restriction, pour générer de l'ADN dans lequel tous les fragments sont de moins de 18 kb ;
(iii) effectuer la résolution dudit ADN ayant subi une restriction au moyen d'une chromatographie capable de séparer des fragments, dans la gamme de 0,1 à 18 kb, pour générer une empreinte génétique distincte ;
(iv) comparer ladite empreinte génétique de l'étape (iii) avec l'empreinte génétique d'un sous-type génétique spécifique, dans laquelle les différences dans lesdites empreintes génétiques garantissent une différenciation des sous-types génétiques au sein d'une population d'organismes génétiquement apparentés.

2. Procédé selon la revendication 1, dans lequel ledit organisme est choisi dans le groupe comprenant les procaryotes et les eucaryotes microbiens.

3. Procédé selon la revendication 2, dans lequel ledit organisme est choisi dans le groupe comprenant les bactéries Gram positives et les bactéries Gram négatives.

4. Procédé selon la revendication 3, dans lequel ledit organisme est choisi dans le groupe comprenant les *Escherichia, Salmonella, Shigella, Vibrio, Listeria, Staphylococcus, Streptococcus, Entérocoques, Klebsiela, Nicericia, Pseudomonas* et *Campylobacter.*

5. Procédé selon la revendication 4, dans lequel ledit organisme est choisi dans le groupe comprenant *E. coli* 0157 et *Salmonella.*

6. Procédé selon la revendication 1, dans lequel ledit moyen de chromatographie de l'étape (iii) est choisi dans le groupe comprenant une électrophorèse sur gel, une HPLC, une cytométrie de flux et une chromatographie sur membrane.

7. Procédé selon la revendication 6, dans lequel ledit procédé d'électrophorèse sur gel est choisi dans le groupe comprenant une électrophorèse sur gel capillaire, une électrophorèse sur gel en champs pulsés, une électrophorèse sur gel bidimensionnelle et une électrophorèse en champs inversés.

8. Procédé selon la revendication 7, dans lequel ledit gel est un gel d'agarose ayant une concentration en agarose de 0,6% environ à 2% environ.

9. Procédé selon la revendication 7, dans lequel ledit gel est du polyacrylamide.

10. Procédé selon la revendication 7, dans lequel ledit ADN ayant subi une restriction est coloré avec un colorant de liaison à l'ADN choisi dans le groupe comprenant le bromure d'éthidium, l'iodure de propidium et les colorants de cyanine fluorescente.

11. Procédé selon la revendication 1, dans lequel au moins ladite enzyme de restriction est choisie dans le groupe comprenant les *AccI, AluI, AvaI, AvaII, BanII*, *BfaI*, *CfoI, DcleI*, *DpnI, HaeII, HaeIII, HhaI, HincII*, *HinfI, HpaII*, *MboI, MnII*, *MseI, NciI*, *NlaIV, RsaI*, *Sau3AI, ScrfI*, *TaqI* et *ThaI*.

12. Procédé selon la revendication 1, dans lequel au moins ladite enzyme de restriction est une coupeuse de 4 bases.

13. Procédé selon la revendication 1, dans lequel au moins ladite enzyme de restriction est une combinaison d'au moins deux coupeuses de 6 bases.

14. Procédé selon la revendication 1, dans lequel ledit ADN génomique est de 5 µg environ à 30 µg environ.

15. Procédé selon la revendication 1, dans lequel ladite résolution de l'ADN, ayant subi une restriction dans l'étape (iii), conduit à l'élimination de tous les fragments d'ADN de moins de 2 kb.

16. Procédé pour la différenciation entre les souches d'isolats bactériens, comprenant les étapes de :
(i) isoler l'ADN génomique à partir : a) d'un panel d'isolats bactériens reliés d'un point de vue épidémiologique ; et b) d'un panel d'isolats bactériens non reliés d'un point de vue épidémiologique, dans lequel lesdits isolats reliés et non reliés sont membres d'un même genre ;
(ii) effectuer une restriction de l'ADN génomique provenant des panels d'isolats reliés et non reliés avec un panel d'enzymes de restriction choisi dans le groupe comprenant les *AccI, AluI, AvaI, AvaII, BanII, BfaI, CfoI, DdeI, DpnI, HaeII, HaeIII, HhaI, HincII, HinfI, HpaII, MboI, MnII, MseI, NciI, NlaIV, RsaI, Sau3AI, ScrfI, TaqI* et *ThaI* ;
(iii) effectuer la résolution de l'ADN de l'étape (ii), ayant subi une restriction, au moyen d'une électrophorèse sur gel pour générer un modèle de bandes pour chaque enzyme ;
(iv) sélectionner au moins une enzyme du panel d'enzymes de l'étape (ii) qui : a) effectue la restriction de tous les ADN génomiques en fragments de moins de 18 kb ; b) génère le plus bas indice de diversité à partir du panel des reliés et c) génère l'indice de diversité le plus élevé à partir du panel des non reliés ;
(v) effectuer la restriction de l'ADN isolé à partir des panels d'isolats bactériens reliés et non reliés de l'étape (i) avec au moins une enzyme de l'étape (iv) ;
(vi) effectuer la résolution dudit ADN de l'étape (v), ayant subi une restriction, au moyen d'une électrophorèse sur gel capable de séparer des fragments dans la gamme de 0,1 à 18 kb pour générer une empreinte génétique distincte pour lesdits isolats reliés et non reliés ; et
(vii) comparer lesdites empreintes génétiques distinctes de l'étape (vi) dans lesquelles les différences dans lesdites empreintes génétiques garantissent une différenciation de contraintes internes entre les souches desdits isolats bactériens.

17. Procédé selon la revendication 16, dans lequel ledit procédé d'électrophorèse sur gel est choisi dans le groupe comprenant une électrophorèse sur gel capillaire, une électrophorèse sur gel en champs pulsés, une électrophorèse sur gel bidimensionnelle, une électrophorèse par inversion et une électrophorèse verticale.

18. Procédé selon la revendication 16, dans lequel ledit gel est un agarose ayant une concentration en agarose de 0,6% environ à 2% environ.

19. Procédé selon la revendication 16, dans lequel ledit gel est du polyacrylamide.

20. Procédé selon la revendication 16, dans lequel ledit ADN ayant subi une restriction est coloré avec un colorant se liant à l'ADN choisi dans le groupe comprenant le bromure d'éthidium, l'iodure de propidium et les colorants de cyanine fluorescente.

21. Procédé selon la revendication 16, dans lequel au moins ladite enzyme de restriction est une coupeuse de 4 bases.

22. Procédé selon la revendication 16, dans lequel au moins ladite enzyme de restriction est une combinaison d'au moins une coupeuse de 4 bases et d'au moins une coupeuse de 6 bases.

23. Procédé selon la revendication 16, dans lequel ledit ADN génomique est de 5 µg environ à 30 µg environ.

24. Procédé selon la revendication 16, dans lequel ladite résolution de l'ADN, ayant subi une restriction dans l'étape (iii), conduit à l'élimination de tous les fragments d'ADN de moins de 2 kb.

25. Procédé selon la revendication 16, dans lequel ladite résolution de l'ADN, ayant subi une restriction dans l'étape (iii), conduit à la séparation de fragments d'ADN sur une distance de 4 cm environ à 12 cm environ de gel.

26. Procédé selon la revendication 16, dans lequel ledit isolat bactérien est choisi dans le groupe comprenant les *Escherichia*, *Salmonella, Shigella, Vibrio, Listeria, Staphylococcus, Streptococcus, Entérocoques, Klebsiela, Nicericia, Pseudomonas* et *Campylobacter*.

27. Procédé selon la revendication 16, dans lequel ledit isolat bactérien est choisi dans le groupe comprenant *E*. *coli* 0157 et *Salmonella.*
